# EUROPEAN PATENT APPLICATION

(11) **EP 2 782 054 A1**
(43) Date of publication of application: **24.09.2014**
(21) Application number: 13159808.8
(22) Date of filing: 18.03.2013
(51) Int. Cl.: G06Q 10/06, G06Q 50/24

(54) **Personalised medicine system for context based advisory information**

(71) Applicant: Optimal Medicine Ltd, London EC3V 3ND (GB)
(72) Inventor: Munro, Janet, 34380 St Martin de Londres (FR); Turner, Rick, Manchester M1 6DE (GB)
(74) Representative: Miller Sturt Kenyon

(57) **Abstract**

A personalised medicine system comprising: a processor; memory; an input module; and an output module, said personalised medicine system using a knowledge base and being adapted to display pages, each page being associated with a stage in a workflow for guiding a clinical consultation, wherein: for a plurality of said pages, advisory information based on said knowledge base is displayed depending on the stage in the workflow associated with the page. A method and a computer program are also provided.

## Description

### Field of the Invention

The present invention relates to a personalised medicine system, such as a system that provides clinical decision support and in particular relates to a system that provides advisory information based on the context in which the advisory information is provided.

### Background of the Invention

Clinical decision support systems are known and such systems can provide recommendations to clinicians, for example for treatment of a patient. In general, however, such recommendations are not context specific and may not be updated as patient data changes or as treatment of the patient progresses. Accordingly, there is a danger that a clinician is not provided with advisory information at a time when it is useful, so no action is taken based on the advisory information, and that what action is taken is out of date and not tailored to a specific patient.

### Summary of the Invention

The present invention seeks to address these shortcomings by providing a personalised medicine system that displays advisory information to a clinician for different steps in a clinical workflow, preferably based on the most up to date patient information.

According to a first aspect of the present invention, there is provided a personalised medicine system comprising: a processor; memory; an input module; and an output module, said personalised medicine system using a knowledge base and being adapted to display pages, each page being associated with a stage in a workflow for guiding a clinical consultation, wherein: for a plurality of said pages, advisory information based on said knowledge base is displayed depending on the stage in the workflow associated with the page.

Preferably, the knowledge base comprises a plurality of sources, and a plurality of items of advisory information based on different said sources may be displayed for a said page.

In this case, it is preferred that a user is able to select the source on which the advisory information is based.

Preferably, the system is further adapted to use patient information, the displayed advisory information being further based on the patient information.

In this case, it is preferred that the patient information includes a time, the displayed advisory information being further based on a period elapsed since the patient information time.

It is also preferred that the patient information includes patient profile data, the displayed advisory information being further based on the patient profile data.

It is also preferred that the patient information includes at least one of a rating of symptoms suffered by a patient, a rating of drug side effects suffered by the patient, a rating of the patient's adherence to a treatment regimen, a diagnosis of the patient, physical characteristics of the patient, and a treatment regimen for the patient.

Preferably, the system uses a plurality of rules, said rules determining advisory information to be displayed for a said page.

In this case, it is preferred that the plurality of rules includes at least one scheduling rule, said scheduling rule comprising: what advisory information is to be displayed, a time criterion for displaying the advisory information, a condition for resetting a clock for determining when to display the advisory information, and a page on which to display the advisory information.

In this case, it is preferred that the scheduling rule further comprises a condition for changing a timing of the advisory information based on patient profile data.

It is also preferred that the system is further adapted to display at least one scheduling rule and to receive an input from a user for changing the time criterion.

Preferably, at least one said rule for a said page comprises a hierarchy of conditions, each condition being connected with at least one other condition in the hierarchy, said patient information being used to establish whether conditions within the hierarchy are met to determine advisory information to be displayed.

Preferably, the system is adapted to: determine the context in which the advisory information is to be provided; determine evolving treatment history of the patient; determine patient characteristics; and depending on the determinations, provide the advisory information.

According to another aspect of the present invention, there is provided a method of providing advisory information in a personalised medicine system comprising: providing access to a knowledge base; providing access to patient information; providing access to rules; displaying pages, each page being associated with a stage in a workflow for guiding a clinical consultation; and, for a plurality of said pages, displaying advisory information depending on the stage in the workflow associated with the page, said advisory information being based on at least one said rule, said knowledge base and the patient information.

According to another aspect of the present invention, there is provided a computer program stored on a computer-readable storage medium for causing a computer to carry out a method of providing advisory information in a personalised medicine system comprising: providing access to a knowledge base; providing access to patient information; providing access to scheduling rules; displaying pages, each page being associated with a stage in a workflow for guiding a clinical consultation; and for a plurality of said pages, displaying advisory information depending on the stage in the workflow associated with the page, said advisory information being based on at least one said rule, said knowledge base and the patient information.

### Brief Description of the Drawings

Embodiments of the present invention will now be described by way of further example only and with reference to the accompanying drawings, in which:
Fig. 1 is a schematic diagram of the architecture of a device for use in the present invention;
Fig. 2 is a schematic diagram of a system according to the present invention;
Fig. 3 is a table representing a workflow for use in the present invention;
Fig. 4 is a partial representation of a screen for display in the present invention, including a header, another display page and a guidelines area;
Fig. 5 is a partial representation of an actions from last visit display page for use in the present invention;
Fig. 6 is a partial representation of an actions due display page for use in the present invention;
Fig. 7 is a partial representation of another display screen for use in the present invention, including two display pages and a guidelines area;
Fig. 8 is a partial representation of another display screen for use in the present invention, including a display page and a guidelines area;
Fig. 9 is a partial representation of another display screen for use in the present invention, including a header, a display page and a guidelines area;
Fig. 10 is a partial representation of another display screen for use in the present invention, including a header, a display page and a guidelines area;
Fig. 11 is a representation of a pop-up display for a symptom rating scale for use in the present invention;
Fig. 12 is a partial representation of a symptom severity sliders display page for use in the present invention;
Fig. 13 is a partial representation of side effect rating scale display page for use in the present invention;
Fig. 14 is a partial representation of another display screen for use in the present invention, including a header, a display page and a guidelines area;
Fig. 15 is a partial representation of a side effect severity sliders display page for use in the present invention;
Fig. 16 is a partial representation of another display screen for use in the present invention, including a header, a display page and a guidelines area;
Fig. 17 is a partial representation of another display screen for use in the present invention, including a header, a display page and a guidelines area;
Fig. 18 is a partial representation of a side effect relative risk display page for use in the present invention;
Fig. 19 is a partial representation of a drug information display page for use in the present invention;
Fig. 20 is a schematic diagram of an advisory information system for use in the present invention;
Fig. 21 is a schematic representation of hierarchical rule for use in the present invention;
Fig. 22 is an exemplary extract of a monitoring rules table for use in the present invention;
Fig. 23 is a partial representation of a monitoring schedule display screen for use in the present invention;
Fig. 24 is a flow chart illustrating a rule for use in the present invention.

### Detailed Description

### General Architecture

The present invention provides a personalised medicine system, which may be a specialised electronic device having only the function of the personalised medicine system. More usually the personalised medicine system of the present invention is an existing electronic device, such as a computer, adapted to have the functionality required by the present invention. Moreover, the personalised medicine system of the present invention may be distributed between several electronic devices/computers which are connected by one or more of a wired LAN, a wireless LAN, a WAN and the Internet.

Where the personalised medicine system is embodied as single electronic device it may be an existing mobile communications device such as a smart phone (for example iPhone^{™} or Android^{™} mobile/cell phone) or a tablet computer (for example iPad^{™} or Samsung Galaxy^{™} tablet). Such a mobile communications device can be adapted to have the functionality of a reminder device according to the present invention by downloading an application or widget to the mobile communications device. In some embodiments, it may interact with other devices to provide the functionality. In others, the mobile communications device may operate in a stand alone fashion.

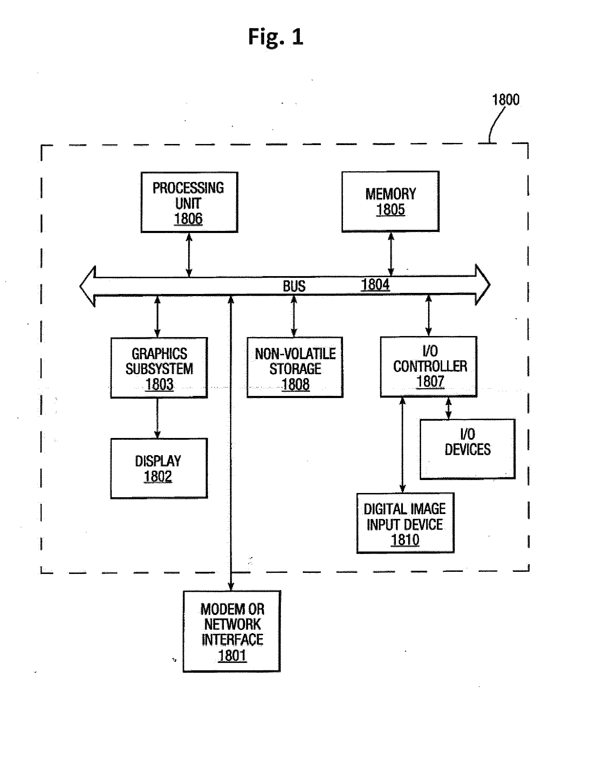

Fig. 1 illustrates an exemplary computer architecture 1800 by which a device embodying or forming part of the personalised medicine system according to the present invention may be implemented. Computer architecture 1800 may be or form part of a desktop computer or a laptop computer, a server, a mobile communications device or any similar computer device.

The computer architecture 1800 may interface to external devices such as another computer, the cloud or Internet, through a modem or network interface 1801, such as an analogue modem, ISDN modem, cable modem, token ring interface, or satellite transmission interface. The network interface 1801 may also be a standard communications module adapted to communicate with a standard mobile communications network, such as 2G, GSM, GPRS, EDGE, 3G, UTMS, CDMA 2000, HDSPA, LTE, 4G, etc networks; a Bluetooth radio; a Wi-Fi radio; or a combination of any two or more of the foregoing.

As shown in Fig. 1, the computer architecture 1800 includes a processing unit 1806, which may be a conventional microprocessor, such as commonly provided by Intel, ARM or AMD, which are known to one of ordinary skill in the computer art. System memory 1805 is coupled to the processing unit 1806 by a system bus 1804. System memory 1805 may be a DRAM, RAM, static RAM (SRAM) or any combination thereof. Bus 1804 couples processing unit 1806 to system memory 1805, to non-volatile storage 1808, to graphics subsystem 1803 and to input/output (I/O) controller 1807. Graphics subsystem 1803 controls a display device 1802, such as a liquid crystal display device, which may be a touchscreen device and/or may be part of the graphics subsystem 1803. The other I/O devices may include one or more of a keyboard, disk drives, printers, a mouse, a speaker, a camera 1810 and the like as known to one of ordinary skill in the computer art.

In one embodiment, the personalised medicine system device 1 is implemented using an application or a widget loaded onto a mobile communications device (such as a smart phone or tablet computer) having a touch screen display device 1802 and a camera 1810 and a speaker (not shown) as I/O devices, and optionally a microphone (not shown) as a further input device. The application causes the device 1 to store predetermined data in the non-volatile storage 1808, which is used to provide the required functionality.

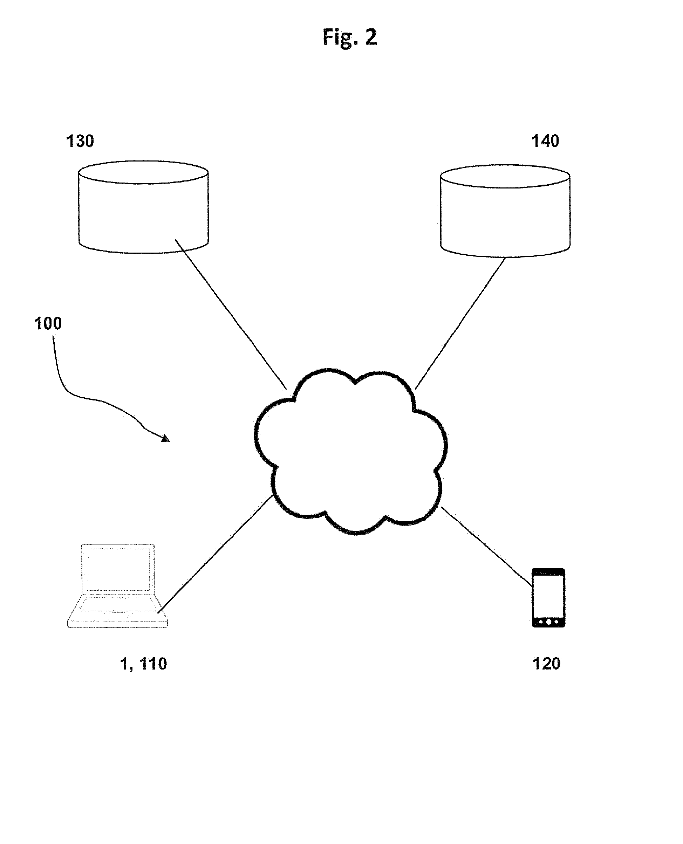

Fig. 2 shows a personalised medicine system 100 comprising a clinician computer 1, 110, a patient smartphone 120, and external servers/computers 130, 140 all of which are connected by one or more of a LAN, a WAN and the Internet. As will be discussed in detail below, the personalised medicine system stores various data, such as workflow information, page information, patient information, slider placement calculation information, side effect severity calculation information, knowledge base information and so forth. Such information may all be stored only on the clinician computer 1, embodying a personalised medicine system of the present invention by itself, or distributed between the clinician computer 110 and one or more networked or remote servers/computers 130, 140, which together embody the personalised medicine system 100 of the present invention. Likewise, processing operations required to provide the functionality of the personalised medicine system 100 may be carried out by the clinician computer 1 alone or may be distributed between the clinician computer 110 and the servers/computers 130, 140. Accordingly, where this specification discusses a particular device such as device 1, 110 storing particular information or carrying out a particular function, it should be understood that in different embodiments the storage and/or functions may be carried out by another device 130, 140 as well or instead.

The patient smartphone, tablet computer or other patient device 120 may provide information to the system 100, such as a record of patient adherence to a treatment regimen, as will be discussed in more detail below.

In some embodiments, multiple clinicians' computers 110 and/or patient devices 120 may be included in the system of the present invention.

### General Operation

The personalised medicine system 1, 100 operates to guide a clinician through a series of interviews with a patient to assist the clinician to gather information about the patient and to decide on appropriate treatment. Thus, the personalised medicine system 1, 100 acts as a clinical decision support system (CDSS) and provides a personalised patient pathway to tailor diagnosis and treatment of an illness or other medical condition to specific patients. In the following description, the personalised medicine system 1, 100 will be described with reference to schizophrenia as an example of an illness/medical condition to which the personalised medicine system is tailored. However, it should be understood that the personalised medicine system 1, 100 can be tailored to other illnesses/medical conditions such as rheumatoid arthritis and cancer, as well as being applied to illnesses/conditions in general.

In order to carry out this functionality, the personalised medicine system 1, 100 stores a plurality of pages for displaying to a user and one or more workflows, each of the pages being associated with a step or node in a workflow. Each node in the workflow has one, two more pages associated with it.

In the present embodiment, the personalised medicine system 1, 100 stores three workflows, Workflow 1 for a first consultation with a patient, where the patient has not previously reported a contact with a psychiatrist or other mental health professional; Workflow 2 for a first consultation where the patient has previously reported a contact with another psychiatrist or mental health professional; and Workflow 3 for a subsequent consultation using the personalised medicine system 1, 100.

When a user (who will generally be a psychiatrist or other clinician) first starts the personalised medicine system 1, 100, for example by clicking an icon on a desktop or tapping an icon on the touch screen display device 1802 of a tablet computer, or navigating their browser to a specific address on a PC or mobile device, the personalised medicine system 1, 100 first displays a welcome screen (not shown). Here the user is able to select an existing patient previously stored in the system 1, 100 or create a new patient. If the user creates a new patient in the system, he is given the option to select whether or not this is patient's first psychiatric contact. Depending on the choices the user makes at this stage, one of the three respective workflows is selected. Each of the three workflows includes a plurality of stages, comprising one or more steps; one or more nodes associated with each step; and one or more pages associated with each node. One or more knowledge bases may be used to populate various pages. Commencement of a workflow starts a "visit", representing a consultation between the clinician and the patient during which information is reviewed and or recorded in the personalised medicine system 1, 100.

As will be discussed in more detail below, users can also access previous visits and use the workflow to move around the visit to review and/or adjust previously recorded information, as well as to add new information.

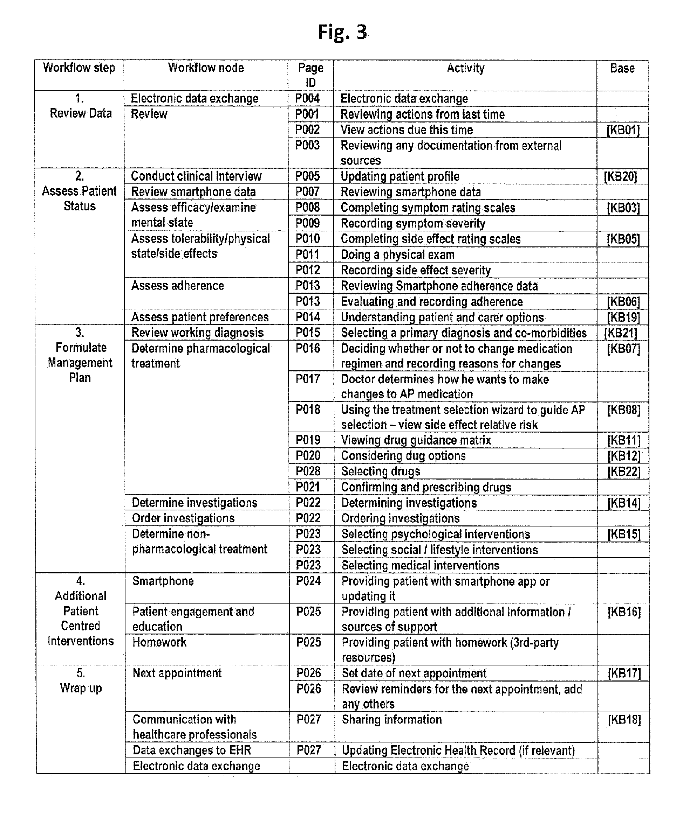

Fig. 3 shows in tabular form Workflow 3 provided for a subsequent consultation by way of example. The other two workflows are similar but changed to take account of their different purposes. The present invention will be described by way of example with reference to the workflow shown in Fig. 3 with relevant changes to the other workflows explained below.

The first workflow step in Fig. 3 for a new consultation with an existing patient is to Review Data and includes two nodes - Electronic Data Exchange and Review. The Electronic Data Exchange node is associated with page ID P004 (not shown). This allows the user to cause the personalised medicine system 1, 100 to interact with other healthcare IT technologies in the clinic/hospital/health authority to carry out automatic data exchange, for example to import patient details stored in electronic health/medical records (EHRs or EMRs) elsewhere, to view previously prescribed and to prescribe drugs, to order lab tests and obtain the results of previously ordered tests. This node may also allow the user to manually enter data, particularly where this is the first psychiatric contact in Workflow 1.

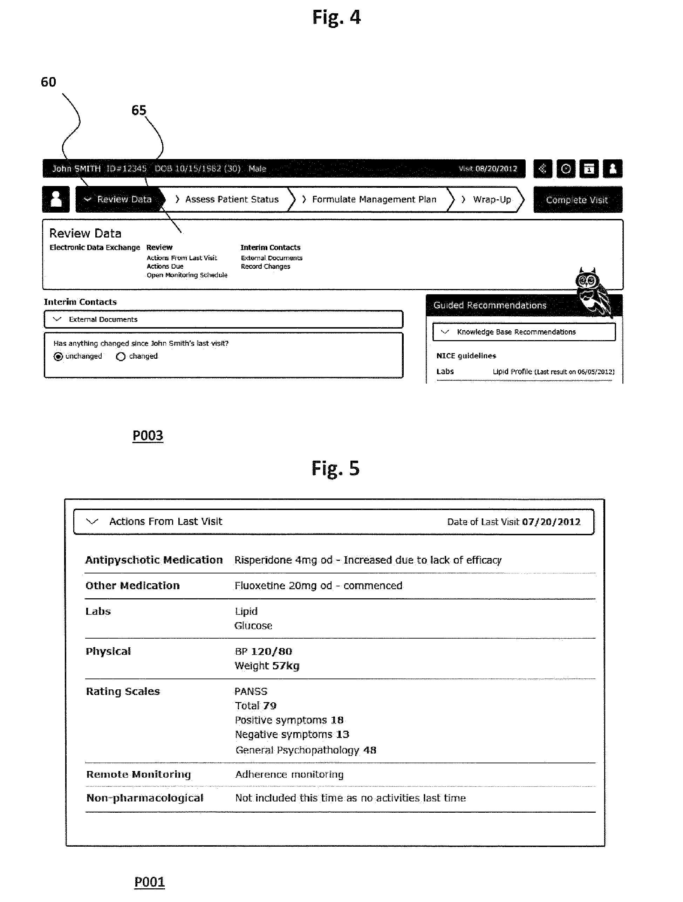

The workflow then moves to the Review node, causing it to display an associated screen. An extract of the screen heading is shown in Fig. 4, which includes the workflow steps in a main heading area 60 and the nodes for the selected step in a sub-heading area 65. The sub-heading area 65 also displays for each node a list of the related activities for which the associated pages are provided. Users can navigate through the workflow by clicking on the respective steps to select them. Once selected, a step is highlighted and a screen sub-heading shows each of the nodes for that step and each of the pages associated with the node. Thus, the user is also able to navigate between nodes and pages by clicking on them to select them. This display structure allows the user to navigate through the workflow in an easy way. Preferably, however, NEXT and PREVIOUS buttons and/or arrows are displayed at the bottom of the screen or elsewhere to allow the user to navigate through a workflow sequentially during a consultation.

In Fig. 4 it can be seen that the user is in the Review Data step of the workflow and is provided with options to enter the Electronic Data Exchange node (discussed above) and the Review node. Within the Review node, the user is able to access pages for reviewing actions from the last visit [page ID P001], viewing actions that are due to be taken this visit [P002] and reviewing documentation from external sources [P003]. In fact, in Fig. 4 the implementation is slightly different from the workflow in Fig. 3 and the option to review documentation from external sources is provided in a separate Interim Contacts node, where it is envisaged that the clinician will view documents created and information changed during an interim contact of the patient with another medical professional since the last consultation. Such interim contacts may occur, for example, because the patient has been admitted to hospital or has obtained psychiatric treatment from another person while on an extended stay at another location. The workflow may be as shown in Fig. 3 or illustrated in Fig. 4.

If the user selects the Review Actions from Last Visit option, for example by clicking on the corresponding wording using a mouse, then page P001 shown in Fig. 5 will be inserted in the screen shown by the display device 1802. Although not shown in Fig. 5, heading area 60 and sub-heading area 65 would also be displayed. Page P001 shows which actions took place as a result of the last visit. Thus, Fig. 5 shows that in the last visit the patient was prescribed risperidone as an antipsychotic medication, fluoxetine as other medication, lipid and glucose labs were ordered, blood pressure and weight were measured, various rating scales were used to assess the patient's symptoms and side effects suffered by the patient, and so forth.

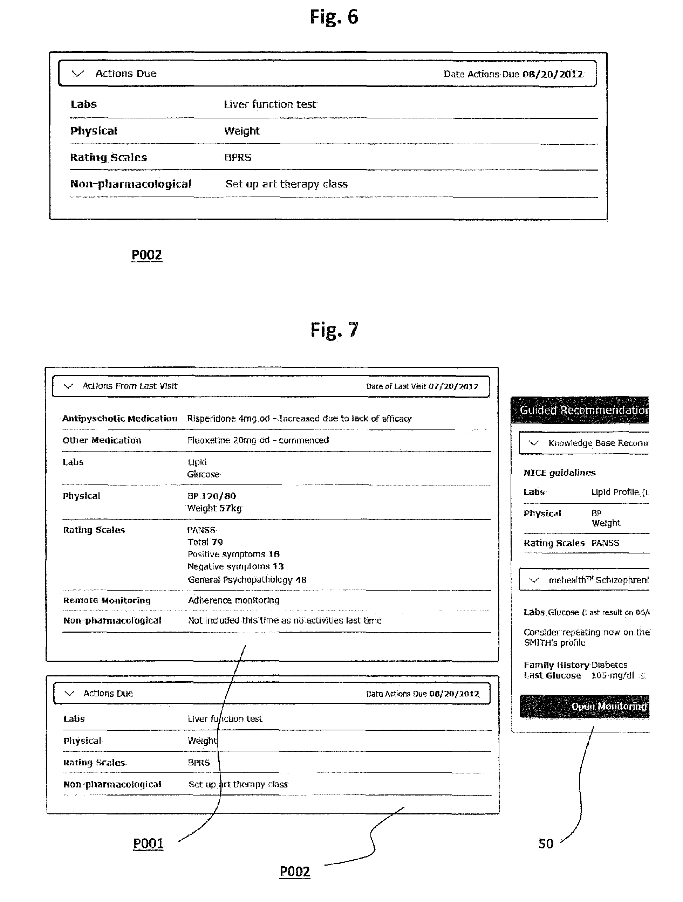

Fig. 6 shows the page P002 by way of further example, which provides the user with a list of actions that should be taken during this consultation. The manner in which this page is populated will be discussed in further detail below.

As illustrated in Fig. 7, in some cases the personalised medicine system 1, 100 may display two or more pages at the same time on the display device 1802. In particular, Fig. 7 shows the concurrent display of pages P001 (actions from last visit) and P002 (actions due). Moreover, as shown in Fig. 7, in addition to displaying the selected pages P001, P002 and P003, the personalised medicine system 1, 100 may simultaneously display guided recommendations for the clinician in guidelines area 50. Again, the population of guidelines area 50 will be discussed in more detail below. As before, heading area 60 and sub-heading area 65 would also be displayed simultaneously.

In workflow 3, following completion of the Review Data step, the workflow moves to the Assess Patient Status step and in particular the first node of conducting a clinical interview. In workflows 1 and 2, since this is the first time the patient has been entered in the personalised medicine system 1, 100, there can be no review of existing data and no due actions will have been set. Accordingly, workflows 1 and 2 omit the Review node of the Review Data step and, following electronic data exchange, move directly to the Assess Patient Status step of the workflow.

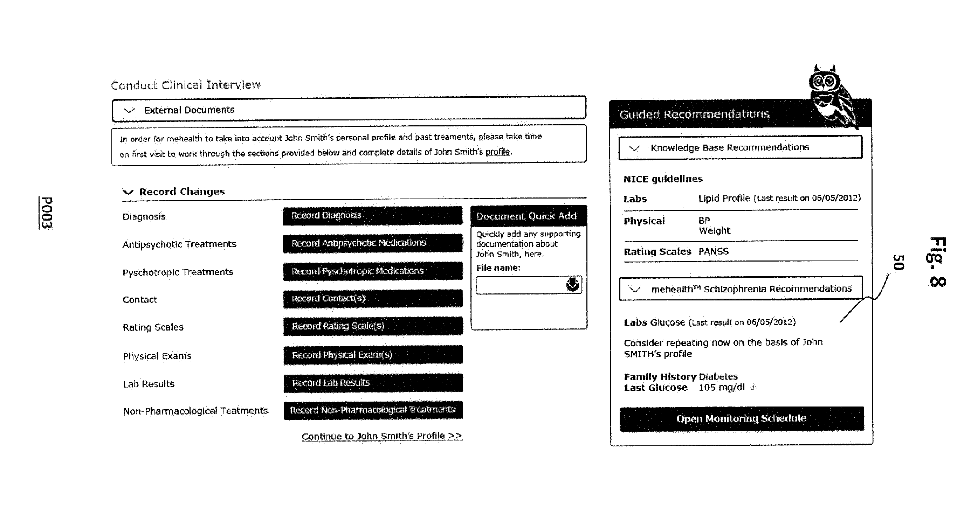

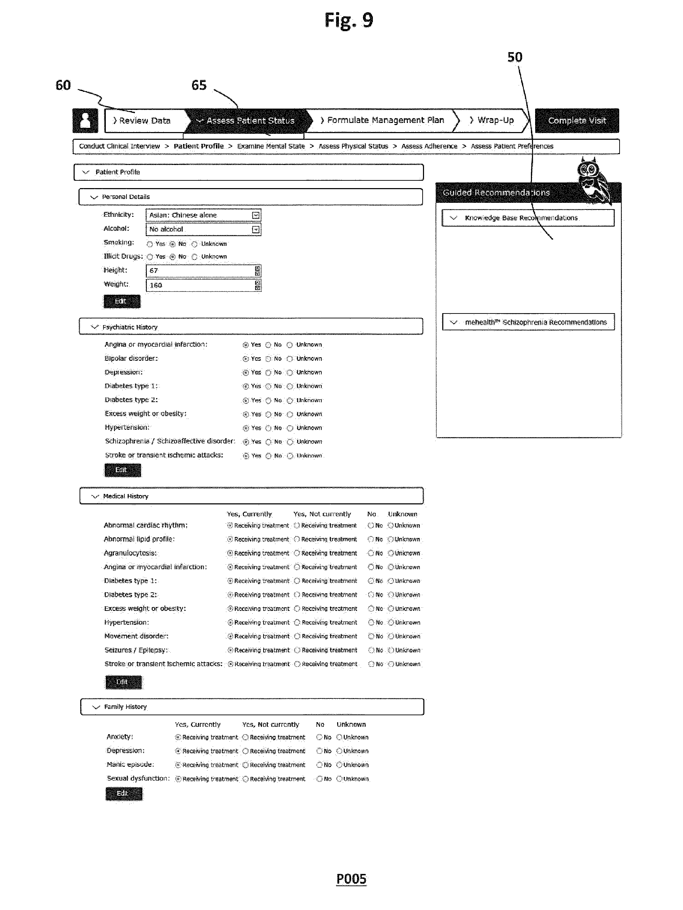

In the Assess Patient Status step of workflows 1 and 2, the first node of conducting a clinical interview is associated with pages P003 (discussed above in respect of workflow 3) and P005. In workflow 3, the conduct clinical interview node is associated only with page P005. Fig. 8 shows an example of page P003 in workflows 1 and 2 together with guidelines area 50. Fig. 9 shows an example of page P005 with guidelines area 50. In workflows 1 and 2, the clinician records the patient profile and clinical history from any available source, including the patient, by systematically working through the prompts in page P003. As shown in Fig. 8, these may include an existing (if any) diagnosis, antipsychotic treatments, other psychotropic treatments, contacts, rating scales, physical examinations, results of laboratory tests, non-pharmacological treatments and so on. Similarly, the clinician completes the patient profile in workflows 1 and 2 or updates the previously-completed patient profile in workflow 3 by following the prompts shown in Fig. 9.

Workflows 1 and 2 then move onto the "examine mental state" node, whereas workflow 3 first allows the clinician to review smartphone data in the "review smartphone" node in conjunction with page P007. In particular, during the (preferably first) visit, the patient's smartphone 120 is updated with an application that allows him to record when he takes his medications and how he is feeling at a given time. The data that has subsequently been input by the patient is then transferred from the smartphone or other patient device 120, for example at predetermined times or during a consultation, onto the clinician's computer 1, 110, and is used to populate page P007 for review by the clinician during the consultation.

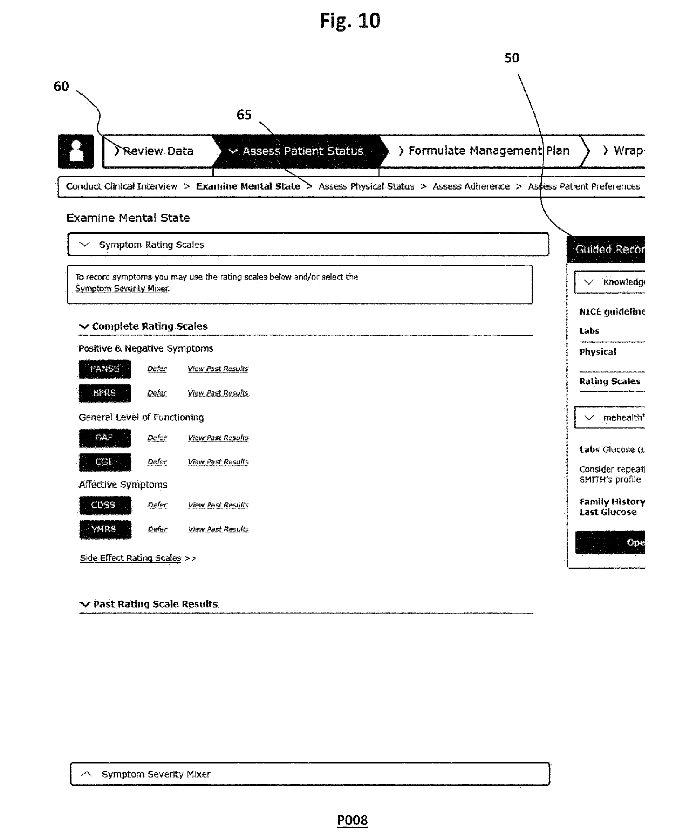

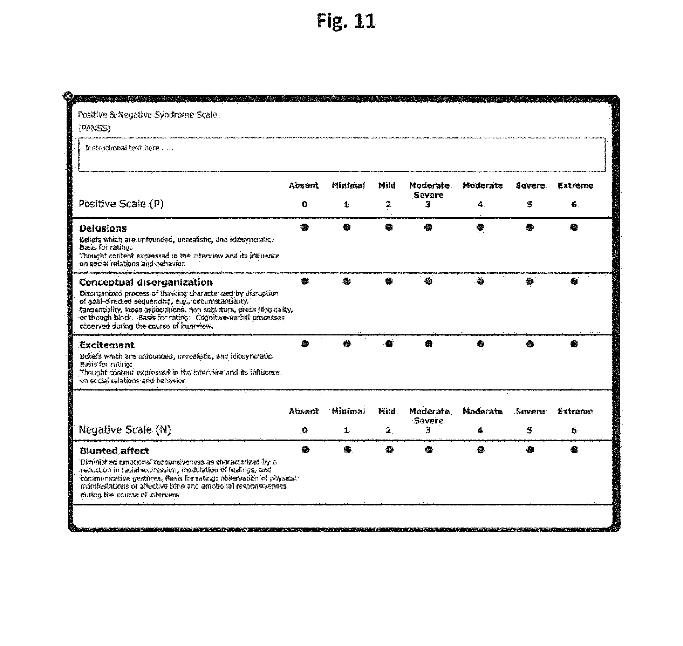

In the "examine mental state" node (which corresponds to the "assess efficacy" node in workflow 3), the user is presented with the option of completing one or more well-established rating scales for assessing the mental state of people suffering psychotic disorders such as schizophrenia. Thus, Fig. 10 shows the display of the screen header 60, 65 highlighting the Assess Patient Status step in the workflow and the "examine mental state" node together with the guidelines area 50. The exemplary symptoms ratings scales shown in Fig. 10 are the positive and negative syndrome scale (PANSS), brief psychiatric rating scale (BPRS), global assessment of functioning (GAF) scale, clinical global impression (CGI) scale, Calgary depression scale for schizophrenia (CDSS) and young mania rating scale (YMRS). However, any number of rating scales may be provided and the user can select any one of the available scales by clicking the corresponding icon to cause a pop-window to open, for example as shown in Fig. 11. The user can then complete the rating scale.

For example, the PANSS is a medical scale used for measuring symptom severity of patients with schizophrenia. To assess a patient using PANSS, an approximately 45-minute clinical interview is conducted. The patient is rated from 1 to 7 on 30 different symptoms based on the interview as well as reports of family members or primary care hospital workers. The symptoms are classified into a positive scale, a negative scale and a general psychopathology scale. The positive sub-scale includes 7 items, minimum score = 7, maximum score = 49 (delusions, conceptual disorganization, hallucinations, hyperactivity, grandiosity, suspiciousness/persecution and hostility); the negative sub-scale includes 7 items, minimum score = 7, maximum score = 49 (blunted affect, emotional withdrawal, poor rapport, passive/apathetic social withdrawal, difficulty in abstract thinking, lack of spontaneity and flow of conversation, and stereotyped thinking) and the general psychopathology sub-scale includes 16 items, minimum score = 16, maximum score = 112 (somatic concern, anxiety, guilt feelings, tension, mannerisms and posturing, depression, motor retardation, uncooperativeness, unusual thought content, disorientation, poor attention, lack of judgment and insight, disturbance of volition, poor impulse control, preoccupation, and active social avoidance).

The other scales operate in a similar manner but include different numbers of symptoms and different scales. For example, the GAF scale is simply a single number selected by the clinician based on his assessment of the patient's overall functioning, based on guidance the scale provides for different number ranges within the scale. Although the different scales may test for different things, generally there is some overlap between scales and different scales may test for (some of) the same things in different ways, for example with different questions and/or different numbers of questions. Other scales may also assess the patient's well-being in different non-clinical domains, for example, quality of life.

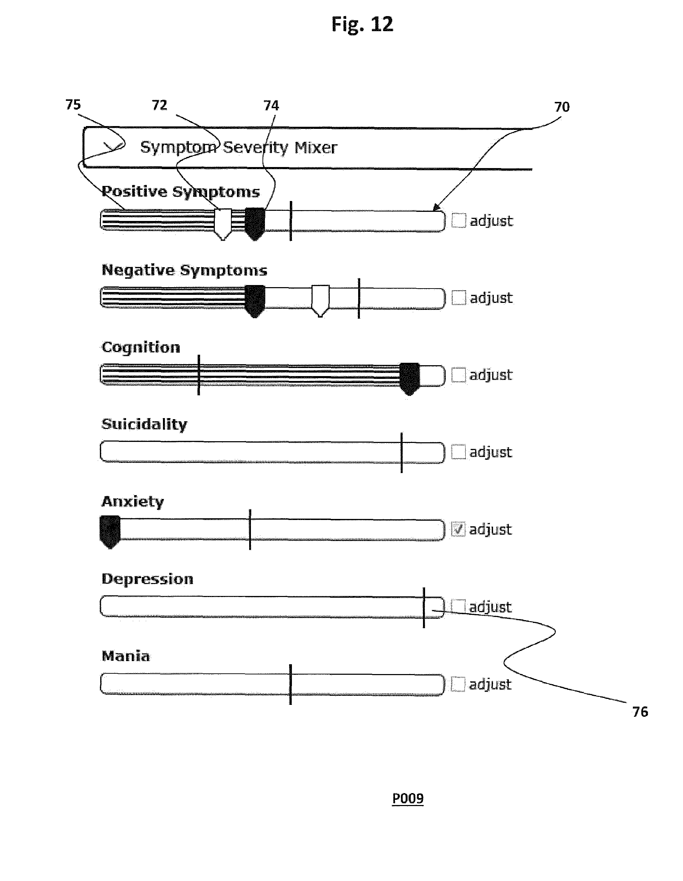

Based on the completion of one or more scales (or none) by the clinician, the personalised medicine system 1, 100 calculates a symptom severity value for each of a plurality of predetermined symptoms and displays the results of the calculations on a graphical scale for each of the symptoms on page P009, as illustrated in Fig. 12. In particular, page P009 shows a graphical representation of linear scale 70 for each of positive symptoms, negative symptoms, cognition, suicidality, anxiety, depression, and mania. Although not illustrated, other scales could also be provided, for example quality of life, and general level of functioning, although this is not an exhaustive list. These graphical representations may be termed symptom (severity) sliders 70 or mixers. In addition to populating the sliders 70 based on the clinician's data input to one or more rating scales in page P008, the user is able to enter a value on the scale based on his own impressions or additional information gained during the visit. Thus, in Fig. 12 shows for each slider 70 a light icon 72 representing a normalised value calculated by the personalised medicine system 1, 100 based on completion of one or more rating scales; a dark icon 74 representing a value enter by the clinician; a fill 75 from 0 up to the dark icon 74 to indicate clearly that the most important value is the clinician updated value; and a line 76 representing the previous value set during the previous visit (which corresponds to the previously set clinician icon 74 or, if the clinician did not input this, the previously calculated normalised value based on completion of one or more rating scales).

Preferably, the user enters a value manually by clicking on the selected slider and dragging an icon along the scale to the appropriate position. If a value has already been calculated by the personalised medicine system 1, 100 or a previous value has been stored, this will be displayed on the slider and the user will be able to use this information together with his experience to position the manually input slider. Alternatively the user may be able to enter a value in box (preferably from 0-10 or 0-100) and this will position the manually input slider on the graphical scale.

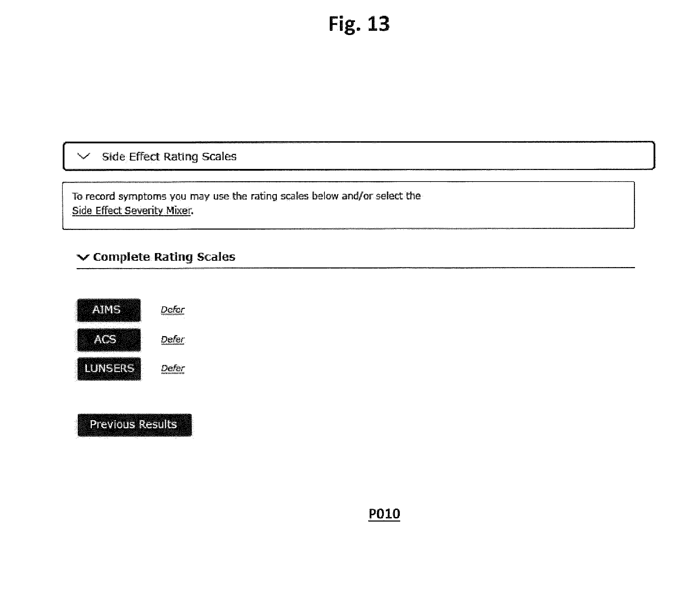

Following completion of the "examine mental state"/"assess efficacy" node, the workflow then moves on to the "assess physical status/side effects"/"assess tolerability' node. This node is associated with page P010, shown in Fig. 13. In this case, the user is given the option to complete one or more predetermined ratings scales to assess side effects of medications experienced by the patient. In the example, the scales are the abnormal involuntary movement scale (AIMS), the antipsychotic side-effect checklist (ASC) and the Liverpool University neuroleptic side effect rating scale (LUNSERS). Again, any number of other rating scales may be provided and again clicking one of the icons causes a pop-up window to open to guide the user through completion of the respective scale.

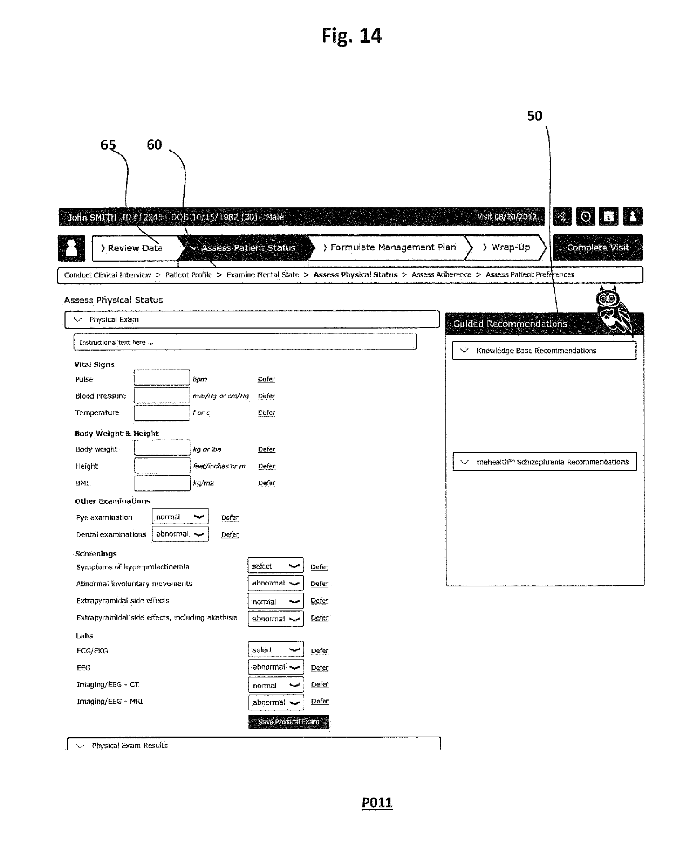

The next page associated with the node is the physical exam page P011. Fig. 14 shows an example of page P011 together with the relevant screen heading 60 and sub-heading 65, as well as the guidelines area 50. As with the other pages, alternative examples are possible. In any event, the user systematically follows the options presented on the page to enter the respective patient details such as height, weight, blood pressure and so forth.

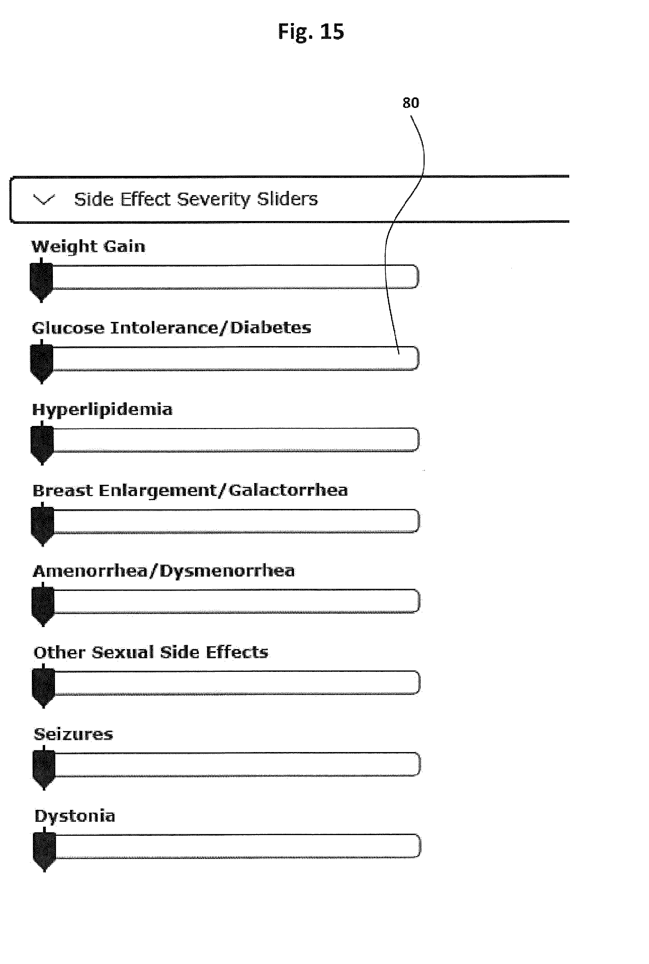

Following completion of pages P010 and P011, the personalised medicine system 1, 100 proceeds to display page P012, shown in Fig. 15, showing side effect (severity) sliders 80. These are populated based upon the completed side effect rating scales and, preferably, the input physical examination data. Similarly to the symptom severity sliders discussed above, the side effect severity sliders include a light icon representing a normalised value calculated by the personalised medicine system 1, 100; a dark icon representing a value manually entered by the clinician; a fill from zero up to the dark icon to indicate clearly that the most important value is the clinician updated value; and a line through the scale representing the previous value set during the previous visit (which corresponds to the previously set clinician icon or, if the clinician did not input this, the previously calculated normalised value based on completion of one or more side effect rating scales).

The user manually enters a value in a side effect severity slider in the same way as discussed above in respect of the symptom severity sliders.

The workflow then moves on to the "assess adherence" node to allow the user to assess how well the patient has adhered to previous or on-going treatment, for example by reviewing data from the patient's smartphone 120 and by checking boxes and entering other information as guided by the corresponding page(s). This node may be omitted or significantly altered in workflows 1 and 2, for example, if the patient is not currently prescribed any medication.

Finally in the Assess Patient Status step, the workflow moves on to the "assess patient preferences" node, where the clinician and patient decide whether to continue with existing medication or to change medication. If a decision to change medication is taken, a pop-up window may open giving the user the option to enter reasons for the change, such as partial efficacy, lack of efficacy or poor tolerability. Again, this node may be omitted or significantly altered in workflows 1 and 2.

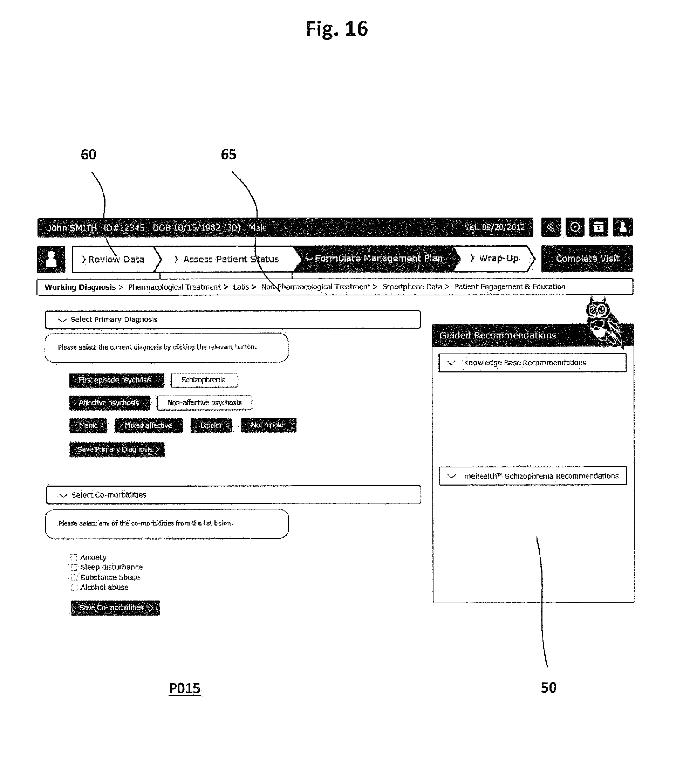

The workflow then moves on to the Formulate Management Plan step. In the first node, "review working diagnosis", page P015 is displayed, as shown in Fig. 16, allowing the user to select or confirm a primary diagnosis and co-morbidities. Preferably, the display of page P015 may change as the primary diagnosis is entered. For example, it may first offer the option of selecting first episode psychosis (FEP) or schizophrenia as the primary diagnosis. If FEP is selected, the page may then offer the selection of affective psychosis or non-affective psychosis. If affective psychosis is selected, the page may then offer the selection of manic, mixed effective, bipolar and non-bipolar. The page also allows the selection of co-morbidities, for example, anxiety, sleep disturbance, drug abuse and alcohol abuse.

In workflows 1 and 2, a "select primary diagnosis" or "determine working diagnosis" node substitute for the "review working diagnosis" node.

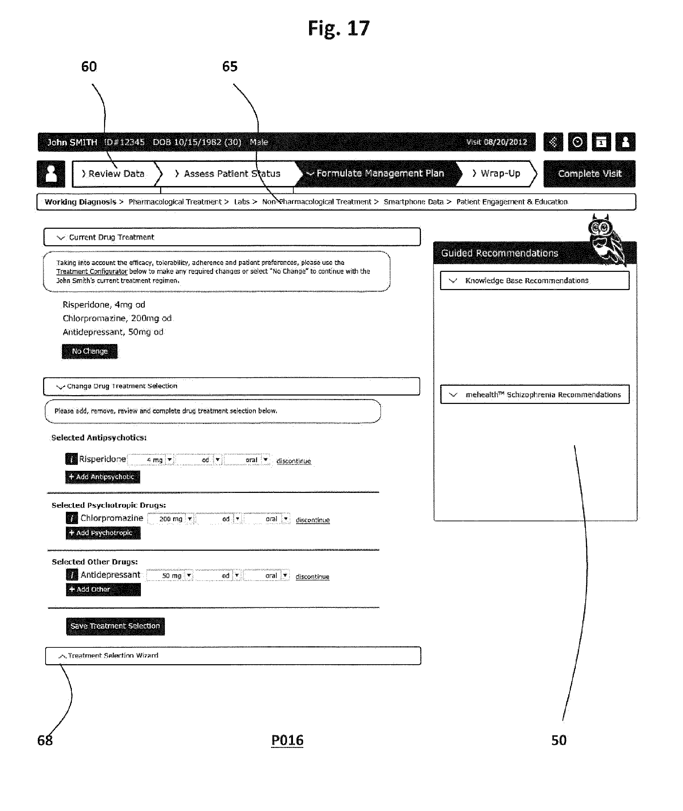

The workflow then moves on to the "determine pharmacological treatment" node, involving the sequential display of pages P016, P017, P018, P019, P020, P028 and P021. Page P016 is shown in Fig. 17 together with heading 60, sub-heading 65 and guidelines area 50. In page P016 the user is shown the current drug treatment regimen and the ability to change the regimen. If the user decides to make a change, pop-up window P017 (not shown) opens requesting the user to select one or more reasons for the decision. As shown in Fig. 17, the user may select one or more new drugs in page P016.

If the user decides not to make any changes, the workflow proceeds to show page P020. In the screen shown on display device 1802, the personalised medicine system 1, 100 provides an option of using an antipsychotic (AP) drug selection wizard or routine provided by the personalised medicine system 1, 100 (see section 68 in Fig. 17) at the same time as showing page P016. If the user decides not to use the wizard, the workflow accepts the changes made in page P016. Otherwise, the workflow moves to page P018.

It will be appreciated that instead of allowing the user to make changes to the drug selection in page P016, an intermediate page may be provided to allow the user either to use the personalised medicine system 1, 100 wizard or to manually select the AP drugs on a new page, which would be similar to the "change drug treatment selection" part shown at the bottom of page P016.

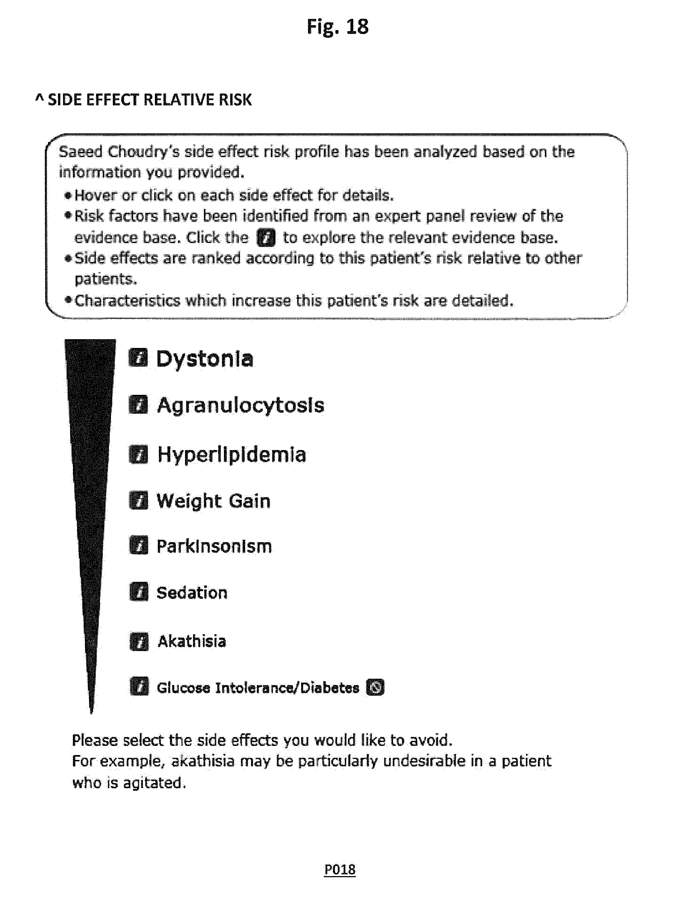

If the user decides to use the wizard, the personalised medicine system 1, 100 may first present, for a plurality of potential side effects, any characteristics which the patient possesses which increase the likelihood of experiencing the side effect. The strength of influence on the side effect of each specific characteristic is presented. The personalised medicine system 1, 100 then calculates, for a plurality of potential side effects, a risk that the patient will experience the side effect relative to the general population. The side effects are then shown in ranked order in page P018, as illustrated in Fig. 18. The manner in which the relative side effect risks and ranked order are determined will be discussed in more detail.

The user may optionally select one or more of the listed side effects which the clinician or patient wants to avoid. For example, a particular patient may particularly want to avoid weight gain or a clinician may recognise that akathisia is undesirable in a patient who is already agitated. A selected side effect may be shown in a different colour, size or font to the other side effects shown on the page.

The user may also hover over or click on each side effect for further details of the side effect, general factors which moderate the side effect, and specific factors extracted from the patient data used in the calculation. The user may also click on an information button next to each side effect to explore the evidence base for the side effect.

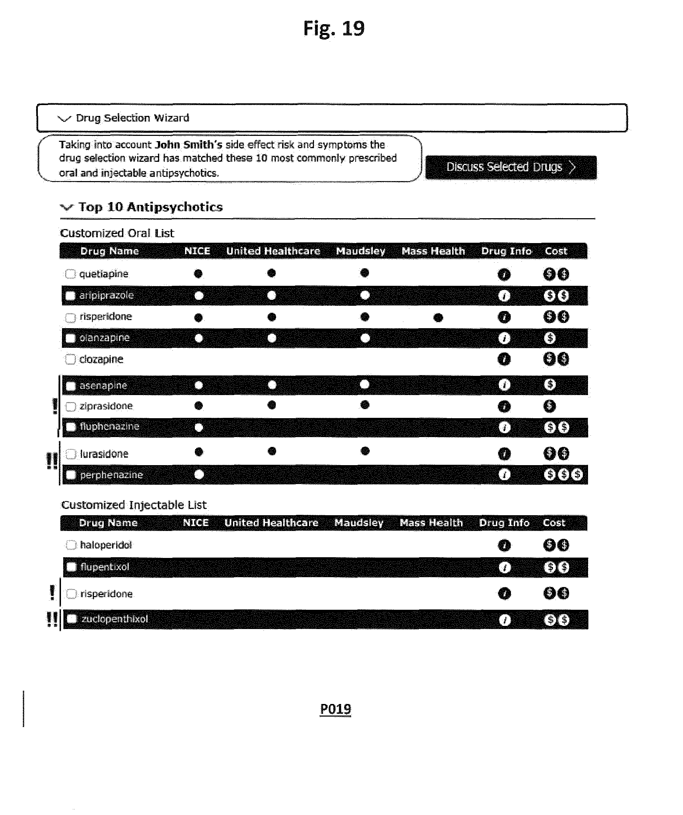

Depending on the working diagnosis selected in page P015, the personalised medicine system 1, 100 then displays in page P019 a list of AP drugs which could be used to treat the patient (see Fig. 19). Moreover, as shown in the figure, the personalised medicine system 1, 100 displays the list of selected AP drugs together with an indication for each drug of whether it is recommended/approved in respective predetermined clinical guidelines by placing a column for each guideline alongside the list of drugs and placing a dot or other marker in each column for each recommended/approved drug. In the example in Fig. 19, the predetermined guidelinesare those published by the UK National Institute for Health and Clinical Excellence (NICE), UnitedHealthcare in the US, the Maudsley psychiatric hospital in the UK, and MassHealth in the US. Naturally, these guidelines are shown by way of illustration only and any other guidelines could be chosen as well or instead.

A column is also provided to allow the user to click on an icon for each drug to obtain further information about the drug, for example from one or more of the guidelines, the manufacturer or other sources. Optionally, the user can click on a dot or other icon under the guideline columns to view information in the respective guideline about the respective drug.

A further cost column is provided showing the relative cost of each drug. Again, clicking on an icon in this column may show the user further information about the costs of the respective drug.

Page P019 further includes an indicator (shown on the left hand side in Fig. 19) of the relative risks of each of the displayed drugs to the particular patient, depending on the side effect relative risks calculated by the personalised medicine system 1, 100, a side effect selected to be avoided in page P018 and, optionally, other patient data. In particular, in the example in Fig. 19, ten drugs to be taken orally are displayed with a single exclamation mark (!) placed against drugs 6-8 in the list and two exclamation marks (!!) placed against drugs 9 and 10. Similarly, five drugs to be taken by injection are displayed with a single exclamation mark (!) placed against drug 4 in the list and two exclamation marks (!!) placed against drug 5. A single exclamation mark may be used, for example, to indicate that the drug should be used with some caution, as the patient's profile suggests that he may have an increased risk of certain adverse side effects. Two exclamation marks may be used to indicate that the drug should be used with extreme caution, as the patient's profile suggests that he is likely to have an increased risk of adverse side effects. Clicking on an icon adjacent the drug may provide further information about what the adverse side effects are and the relative risk of experiencing them. The number of exclamation marks also takes into account the views of the patient and doctor about which side effects would be most undesirable.

Accordingly, the drug table provides a wealth of information tailored to the specific patient to allow the clinician and the patient to discuss suitable AP drug treatment options and select an AP drug for treatment.

Irrespective of whether the pharmacological treatment has been changed, the personalised medicine system 1, 100 then shows page P020 (not shown) which is populated with information about the selected AP drug, allowing further discussion of the treatment options. Subsequently, page P028 (not shown) is displayed, allowing the user to select non-antipsychotic psychotropic medications in addition to the selected AP drug. Optionally, page P028 is only displayed the first time an AP drug has been selected/prescribed or if the AP drug selection has changed. The workflow then moves on to shown page P021 (not shown), in which the user confirms and prescribes the selected drug(s).

The workflow then proceeds to the "determine investigations" node to determine which investigations should be carried out on the patient based on the prescribed drug(s), the patient's status, and other information (such as the evidence base and the guideline recommendations, which are stored in a knowledge base 260 discussed below), to obtain baseline values or follow-up evaluations of the patient's tolerability and physical health status; the "ordering investigations" node to order the determined investigations; the "selecting psychological interventions" node for selecting and ordering interventions such as family therapy, cognitive behavioural therapy (CBT) etc; the "selecting social/lifestyle interventions" node for selecting and ordering interventions such as smoking cessation support, gym referral, supported employment etc; and the "selecting medical interventions" node for selecting and ordering interventions such as dietary advice, alcohol/drugs therapy, diabetologist referral etc.

Finally, the workflow moves on to the Additional Patient Centred Interventions and Wrap-up steps of the workflow with their associated nodes and pages.

Accordingly, it will be appreciated that the personalised medicine system 1, 100 of the present embodiment provides a platform written specifically for clinicians and patients in the area of psychiatry, which addresses the unique needs of practitioners as they relate to patient tracking, clinical decision support, clinical references and guidelines, evaluation (treatment and outcome assessment), patient-facing support, and personalised remote self-monitoring. The personalised medicine system 1, 100 provides personalised best practice clinical management decisions by combining industry-recognised guidelines, a published evidence base, a clear workflow for the clinician to follow and the patient's unique characteristics. The present invention improves the care of individuals with chronic mental illness, where there is a large number of different treatments available, each with unpredictable efficacy and troublesome side effects. In addition to providing guidance on the selection of appropriate drugs for treatment of the patient, the personalised medicine system 1, 100 of the present invention also helps the doctor with making non-drug treatment decisions; obtaining, providing and reviewing patient information; monitoring, recording and presenting outcome etc.

Although the present embodiment relates to a personalised medicine system 1, 100 specifically adapted to the treatment of mental illness and, specifically, schizophrenia and other psychosis, it will be apparent that the personalised medicine system 1, 100 of the present invention can be adapted to a host of other illnesses, including especially those illnesses and conditions that require long term care and multiple consultations between patient and physician, such as various different forms of cancer, rheumatoid arthritis and so on. However, the personalised medicine system 1, 100 of the present invention can also be adapted to provide a workflow for the general practitioner or trained nurse to follow in more general consultations in less specialised fields of practice.

In each case, the personalised medicine system 1, 100 of the present invention can provide point-of-care decision support for a wide spectrum of clinical decisions from diagnosis to chronic disease management. It accompanies the doctor and patient across the full continuum of care and throughout the illness, informing therapeutic decisions, monitoring outcomes and offering patient-focused interventions. In doing so, the personalised medicine system 1, 100 allows clinicians to apply the evidence-base to each individual patient and facilitates concordance of the treatment with selected guidelines, promoting consistently higher quality care. Preferably, it can be customised by healthcare providers and doctors to preferred guidelines or formularies.

Specific aspects of the personalised medicine system 1, 100 will now be described in more detail.

### Context Specific Information

As previously discussed the personalised medicine system 1, 100 may display on screen, together with the page(s) associated with the relevant node of a step in the workflow, a guidelines area 50. In addition, as shown in Fig. 6, page P003 shows actions due in the current visit. Other such prompts may appear elsewhere in the workflow. Together, the guidelines area 50 and the other prompts due provide the user with context specific advisory information for the user to follow or review as desired. The provision of the context specific advisory information (guidelines area 50 and the actions due prompts) will now be discussed in more detail.

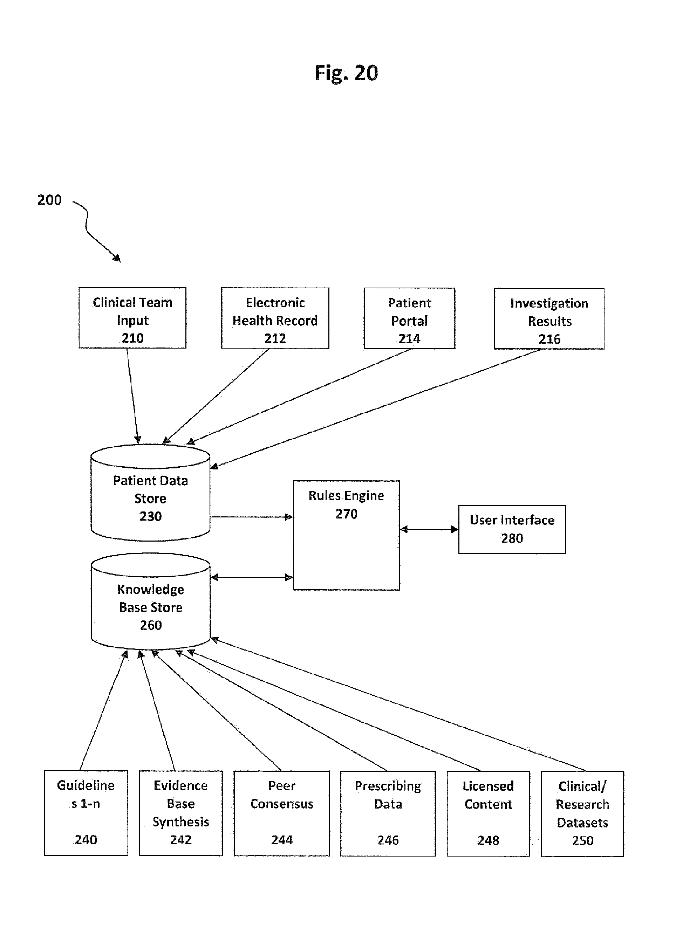

Fig. 20 is a schematic diagram of the advisory information sub-system 200 contributing to the advisory information functionality of the personalised medicine system 1, 100. Specifically, advisory information sub-system 200 comprises a patient data store 230 and a knowledge base store 260.

The patient data store 230 is populated for each patient with information about that patient derived from various sources, such as clinical team input 210; existing electronic health/medical records (EHR/EMR) for the patient 212; a patient portal 214 which receives inputs from the patient in clinic or via their mobile communications or other device 120 (eg smartphone or tablet) for example concerning their mood or taking of medications (including remote monitoring of the patient); and investigation results 216 for example from labs or results of personalised medicine tests, for example for genetic markers.

The knowledge base store 260 derives from inputs from various sources, including various different published clinical guidelines and treatment algorithms 240 (for example, from NICE, UnitedHealthcare and MassHealth); a synthesis of the evidence base 242 relating to the condition to which the personalised medicine system 1, 100 is tailored (for example, mental illness); peer consensus 244; predetermined prescribing data 246; licensed content (for example, a clinical textbook such as the Maudsley Prescribing Guidelines) 248; and clinical/research datasets 250 including de-identified patient data from the system. Known knowledge capture and data mining processes may be carried out on these sources to extract relevant information and to populate the knowledge base. Such processes may be carried out automatically using a computer program to parse and interrogate each knowledge source or by a human, or more usually by a combination of automatic and manual operations.

For example, US patent number US 6,868,422 discloses a method of knowledge acquisition in expert systems suitable for use in the present invention in which data is collected into a computer entity by compiling a set of knowledge data statements, each knowledge data statement representing an element of a knowledge domain; creating lattice data comprising a plurality of nodes with connections between the nodes, each node representing a knowledge data statement, the nodes being arranged in a hierarchical structure and the connections comprising logical connectivity relationships; and creating a consistency matrix, which comprises an array of data entries representing consistency relationships between the knowledge data statements. The data statements can be elicited by using a questioning structure determined from an underlying mathematical base. The contents of US 6,868,422 are incorporated herein by reference.

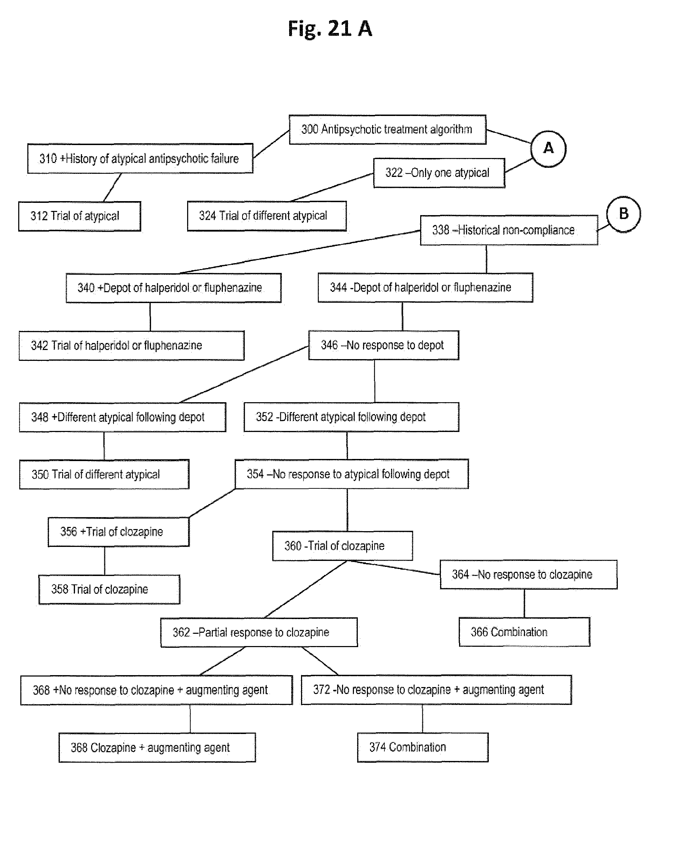

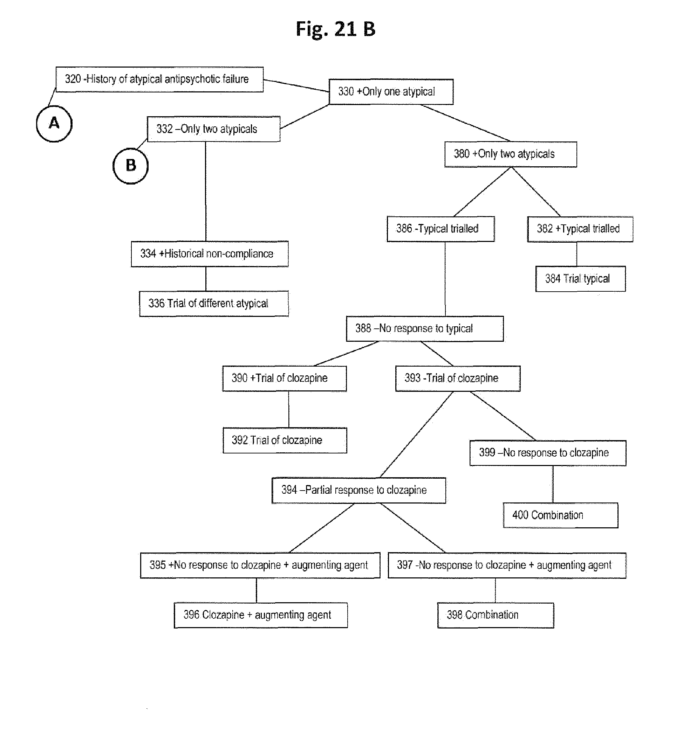

The knowledge base store 260 further stores a plurality of rules for providing the relevant information to the user interface 280 at the relevant time. An example of such a rule is shown in Fig. 21, which provides an antipsychotic treatment rule. The rule comprises a number of knowledge statements, each representing a node in a hierarchical structure with connections between the nodes representing consistency logical relationships between the nodes. In Fig. 21, the root node 300 has two branches leading to nodes 310 and 320, which query whether there has been a history of failure of treatment by atypical antipsychotic (AP) drugs. If there has been no history of treatment failure the network is followed to node 310 and then to node 312, which recommends trialling an atypical AP drug.

By contrast, if there has been a history of failure, the network is followed to node 320 and from there to node 322 if only one atypical AP drug has previously been tried, in which case node 324 suggests trialling a different atypical AP drug.

By contrast, if more than one atypical AP drug has previously been tried, the network is followed to node 330 and from there to node 332 if only two atypical drugs have previously been tried. From node 332 the network is followed to node 334 if there has been no history of non-compliance and to node 338 if there has been a history of non-compliance. If the network is followed to node 334, a recommendation is given in node 336 to trial a different atypical drug.

From node 338 the network is followed to node 340 if a depot neither haloperidol nor fluphenazine have previously been tried, in which case a trial of a depot of one of them is recommended at node 342. If a depot of one of them has previously been tried (node 344) and there has been no response to the depot (node 346), then it is queried whether a different atypical drug has been tried following the depot. If not (node 348), then trial of a different atypical drug is suggested (node 350). If a different atypical drug has been tried following the depot (node 352) and there has been no response (node 354), then it is queried whether clozapine has been tried. If clozapine has not been tried (node 356), the trial of clozapine is recommended (node 358). By contrast, if clozapine has previously been tried (node 360), and there has been a partial response (node 362) and there has not been no response to clozapine with an augmenting agent (node 368), then clozapine with an augmenting agent is suggested (node 370). By contrast, if there has been no response to clozapine with an augmenting agent (node 372), then a combination of clozapine with another atypical AP drug is recommended

(node 374). Alternatively, if clozapine has been tried (node 360) with no response (node 364), then a combination of clozapine with another atypical drug is recommended (node 366).

It is noted that in several instances only a node for "no response" is provided and there is no node for "there has been a response"; or only nodes for "no response" and "partial response" are provided. This is because it is assumed that if there has been a response the drug is working well and there will be no desire to change the pharmacological treatment.

Returning to node 330 (not only one atypical AP drug has been tried), the network is followed to node 380 if not only two atypical AP drugs have been tried. The network is then followed in a similar manner down the various branches until a recommendation is reached at the end of a branch.

This rule would be activated to populate the guidelines area 50 shown alongside page P016 (see Fig. 17), for example.

Naturally, the knowledge base store 260 stores a large number of such rules, together with the underlying data to display in the guidelines area 50 once a rule network was been parsed. Such rules may allow for or even require the input of various different items of patient data or other information to be successfully parsed.

These rules may be grouped into subsidiary knowledge bases, and the various pages may call upon the subsidiary knowledge bases to populate the guidelines area 50 or the advisory action section of the page. Thus, the table in Fig. 3 shows that page P002 in the "review" node of the Review Data step in workflow 3 calls upon subsidiary knowledge base KB01, page P005 in workflow 3 calls upon subsidiary knowledge base KB20, and so on.

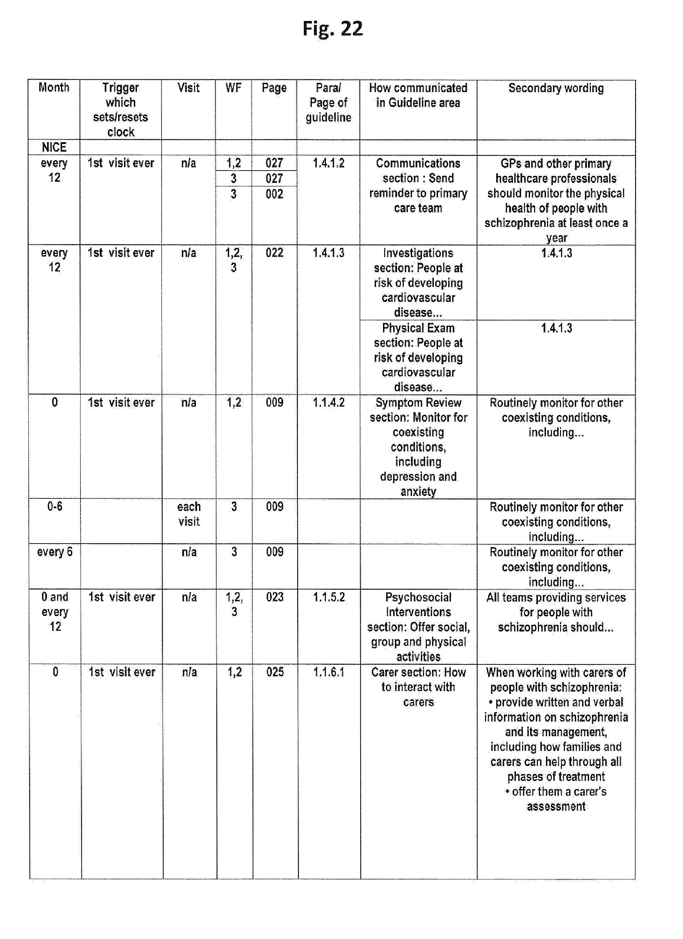

As well as including the subsidiary knowledge bases, the knowledge base store 260 also stores a monitoring rules table, an exemplary extract of which is shown in Fig. 22. In particular, Fig. 22 shows some of the monitoring rules provided for the NICE clinical guidelines included in the knowledge base. However, many more monitoring rules may in practice be provided for this guideline, and an additional set of monitoring rules may be provided for each different guideline. In effect, each guideline is automatically or manually parsed to find each time-based advisory action or item of information and this is incorporated in the monitoring rules table. The monitoring rules table includes columns for a frequency (month - although another unit of time could be selected such as days or weeks); a trigger which sets a clock; a visit; a workflow WF; a page ID; the relevant paragraph or page of the guideline, how the information is communicated to the user in the guidelines area 50; and secondary wording to display on receipt of a request by the user for more information.

For example, taking the first rule in the NICE monitoring table for specific advisory information, the advisory information should be displayed every 12 months starting from the first visit ever. Thus, the information will be displayed in the first consultation between the clinician and patient and the next visit occurring after 12 months has expired. If the visit causes the personalised medicine system 1, 100 to run workflows 1 or 2, the advice is included in the guidelines area 50 when page P027 is displayed. More particularly, the wording "Send primary reminder to care team" is displayed in the Communications section of the page. If the user clicks on this wording or another information icon, the additional wording "GPs and other primary healthcare professionals should monitor the physical health of people with schizophrenia at least once a year", and the user will also have access to the whole NICE guidelines document with the specific wording highlighted.

If the visit causes the personalised medicine system 1, 100 to run workflow 3, the advisory information is included in the guidelines area 50 both when pages P002 and P027 are displayed.

In the second rule, wording is displayed in each of the Investigations section and the Physical Exam section of the "determine investigations" page P022 in each of workflows 1, 2 and 3. If further information is requested, the wording of the appropriate paragraph of the guideline is repeated. As with other pages, it will be appreciated that rules in the knowledge base store 260 other than those included in the monitoring rules table may be used to populate this page, for example based on the drug prescribed for treatment of the patient.

In the third rule, the advisory information is only displayed in the first visit and not in subsequent visits. Accordingly, the rule does not apply to workflow 3.

In the fourth rule, advisory information is displayed each visit for the first six months and is not displayed again afterwards. This rule applies only to workflow 3, so the patient will be on a follow up visit when the advisory information is displayed in page P009.

It will be appreciated that some rules only require the display of particular information in the guidelines area 50 or a predetermined part of a page, without any further conditions are being required. By contrast, other rules may be very complicated and require checking whether a large number of conditions are met before a decision is made on what advisory information, if any, is to be displayed.

The advisory information sub-system further comprises a rules engine 270 lying between the patient data store 230 and the knowledge base store 260 on one side and the user interface 280 on the other side. The rules engine 270 has the function of combining data from the knowledge base store 260 and the patient data store 230 in response to inputs from the user interface 280 and of providing contextualised advisory information to the user interface 280 for display to the user.

Thus, as the user works through a workflow, each time a user calls up a page the rules engine 270 checks whether the page ID is associated with a particular rule in the monitoring rules table or with a particular subsidiary knowledge base. If the page ID is associated with a rule in the monitoring rules table or a particular subsidiary knowledge base, the advisory information associated with the rule is passed by the rules engine 270 to the user interface 280 for display in accordance with the rule.

For example, when page P002 is called up in workflow 3, the rules engine 270 establishes that the first rule in the table in Fig. 20 might apply, checks from the patient data store 230 whether the advisory information associated with that rule was displayed more than 12 months ago and, if it was, passes the wording "Send reminder to primary care team" to the user interface 280 to display in the Communications section of page P002.

An example is shown in Fig. 7, which shows pages P001 and P002 displayed at the same time, together with the guidelines area 50. In this case, the first rule in the table is not invoked because less than 12 months has expired since the first visit and wording "Send reminder to primary care team" is not displayed in the guidelines area 50. However, the guidelines area 50 is caused to display that the NICE clinical guidelines recommend that on this visit lipid profile labs are ordered, blood pressure and weight are measured, and that a PANSS is completed.

Moreover, additional proprietary guidelines included in the personalised medicine system 1, 100 recommend that, for example, a glucose test is ordered on a more frequent basis than the NICE guidelines recommend (and gives the date of the last result available for a glucose test), and gives the reason that, for example, the patient has a family history of diabetes as well as the last glucose level being high.

In this case, a more complicated rule has been used for the proprietary guidelines than the exemplary rules shown in the monitoring schedule table in Fig. 22. In particular, the proprietary guideline uses a rule that checks the family history of the patient, the date of the last test result for glucose and the glucose level of the last test result to determine whether to recommend performing a glucose test. Thus, based on the page ID, the rules engine 270 extracts the relevant rule(s) and any supporting information that may need to be displayed from the appropriate subsidiary knowledge base KB stored in the knowledge base store 260 and any required information from the patient information store 230 required for the extracted rules. The rules engine 270 then runs through each of the extracted rules, using the retrieved information from the patient data store 230 as necessary, to determine if any advisory information should be displayed in the guidelines area 50 and, if so, what. Depending on the result, the rules engine 270 passes the relevant advisory information from the knowledge base store 260 and the patient data store 230 to the user interface 280 for display on the display device 1802. The user may then click on or otherwise select some of the displayed advisory information to cause the user interface 280 to retrieve (if not received with the initially displayed advisory information) the secondary wording.

In general then, the personalised medicine system 1, 100 searches the knowledge base store 260 for information related to the activity being performed (the stage in the workflow - step, node or page) - in another example, similar to the rule shown in Fig. 22, the clinician wants to treat the patient with an antipsychotic medication.

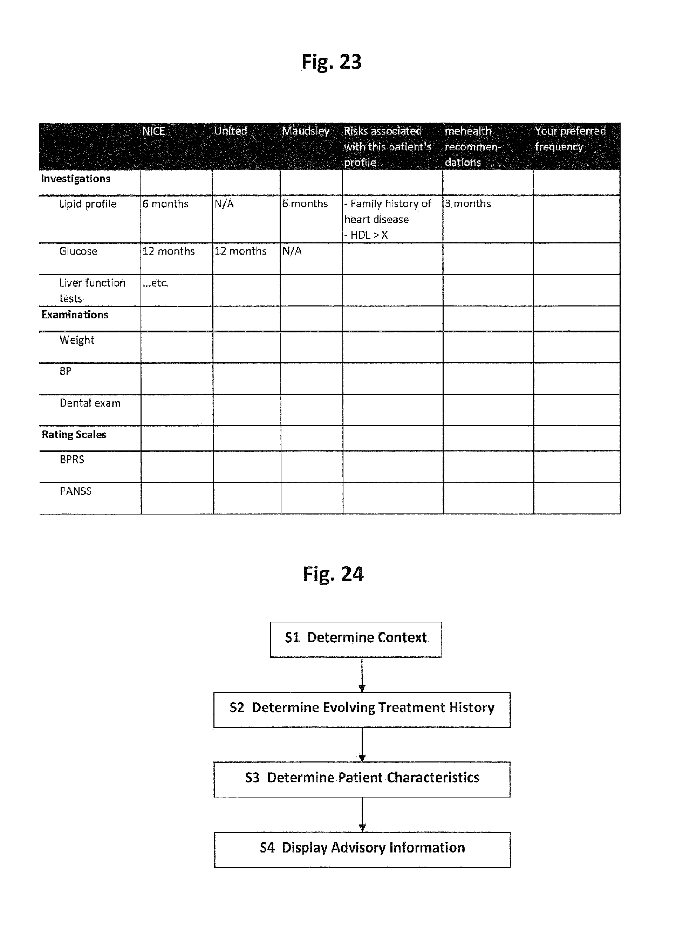

In more general terms, the personalised medicine system 1, 100 examines the context (step S1 in Fig. 24)- in this example, is the working diagnosis first episode psychosis? In this example, NO.

The personalised medicine system 1, 100 then examines the evolving treatment history (step S2 in Fig. 24). In this example, has there been past treatment with 2 or more antipsychotic drugs? YES. Was one of them second generation (ie atypical)? YES. Has there been an adequate trial of the current medication for 4 or more weeks? YES. Has there been a poor response to the current antipsychotic drug? YES. Has clozapine already been trialled? NO.

The personalised medicine system 1, 100 then examines the patient's characteristics (step S3 in Fig. 24). In this example, has there been a history of cardiovascular (CVS) disease? YES.

The personalised medicine system 1, 100 then offers the relevant guidance (step S4 in Fig. 24) - in this example, the guidance is to review diagnosis, evaluate adherence, consider if the current drug has been prescribed at an adequate dose for the correct duration, trial clozapine, do an ECG, discuss the drug with the patient, and record baseline symptom and side effect ratings. This advisory information may all be displayed at the same time, or may be displayed at different relevant times as the workflow progresses. Thus, the recommendation to do an ECG may only be displayed in the guidelines area 50 when the "order investigations" node is reached on the workflow.

It will be appreciated that the information stored and the processes performed may be distributed otherwise than as described above to achieve the same functionality. For example, rather than storing (all of) the advisory information, the knowledge base store 260 may store a link to the appropriate source 240, 242, 244, 246, 248, 250 and retrieve some or all of the required information on demand from the rules engine 270, or the rules engine 270 may retrieve the required information, possibly via the knowledge base store 260.

In addition, the rules engine 270 may receive feedback information from the user interface 280 to adapt the advisory information to be displayed. For example, where the knowledge base store 260 provides advisory information from a plurality of different guidelines (for example, one or more published guidelines and/or a proprietary guideline), the personalised medicine system 1, 100 may enable the user to select the guideline(s) on which advisory information should be based.

It should be noted that the advisory information need not always be displayed in the guidelines area 50. Rather, the advisory information may also be used to populate specific information in one or more pages. For example, page P002 at the bottom of Fig. 7 shows actions due based on rules. It should be noted that in the screen shown in Fig. 7, not only are two pages displayed but the guidelines area 50 also includes further advisory information based on the NICE clinical guidelines and proprietary guidelines.

The personalised medicine system 1, 100 may also allow the user to adapt some of the rules, or how some of the rules are operated. For example, the sub-heading 65 displayed when the main heading 60 highlights the Review Data step in the workflow may include the wording "Open Monitoring Schedule". When this wording is clicked on, the user interface 280 displays a table as illustrated in Fig. 23, which is shown by way of example and is only partially populated. The table shows each of a number of items that should be regularly monitored, such as lab investigations (including lipid profile, glucose, liver function tests); physical examinations (including weight, blood pressure, dental exam); rating scales (including BPRS, PANSS used to monitor symptoms for example) etc. The table further provides a column for each available guideline and shows the frequency of monitoring recommended by each guideline for each item.

In this embodiment, one of the guidelines is a proprietary guideline and the table includes a column showing the risk factors associated with the particular patient's profile that have contributed to the frequency of monitoring recommended for each item by the proprietary guideline based on the rules in the knowledge base store 260 and the data stored in the patient data store 230 for that patient. The user is able to adjust the frequency of monitoring that will be recommended for that patient in the last column. In subsequent operation, the personalised medicine system 1, 100 will provide recommendations for monitoring in the actions due page (P002) based on the proprietary guideline, unless overridden by the user selection, and will provide further advisory monitoring information based on the other guidelines (as selected by the user) and possibly additional information from the proprietary guideline in the guidelines area 50.

Accordingly, the personalised medicine system 1, 100 of the present invention displays context specific advisory information to the user as the user works through a workflow. More particularly, the advisory information displayed is specific to the context of the stage in the workflow (step, node, page) and to the patient data of the patient in consultation. Thus, the personalised medicine system 1, 100 of the present invention provides the clinician with the most appropriate information for that stage, specifically tailored to that patient. Thus, the personalised medicine system 1, 100 drives a higher standard of care than may be expected from a perusal of the guidelines alone.

Moreover, the patient data evolves both as an individual consultation progresses and as the number of visits grows and treatment progresses. The personalised medicine system 1, 100 updates the advisory information displayed as the patient data evolves to ensure the clinician is always presented with the appropriate advisory information in the context of the changed patient data and the stage of the workflow. Because of the way the stages (steps, nodes, pages) in the workflows are ordered, the personalised medicine system 1, 100 can ensure that the appropriate data is gathered before recommendations are made in subsequent stages.

Moreover, as the sources of data 240-250 for the knowledge base store 260 evolve, so the knowledge base store 260 can evolve and be updated. This can occur automatically as the sources 240-250 change. Thus, if the rules lead to display of information from the prescribing data source(s) 246, and the data from the data source changes, this can be reflected in the data shown by the personalised medicine system 1, 100. For example, updates in the prescribing data 246 can be automatically imported into the knowledge base store 260, or the knowledge base store may provide a link to the same place, and the information at the link will change as the source evolves. Naturally, this applies to the other sources of data 240-250.

Moreover, the rules in the knowledge base store 260 may be updated automatically or manually too.

From the foregoing discussion of the general architecture, it will be apparent that each of the patient data store 230, the knowledge base store 260 and the rules engine 270 can be stored on the clinician's computer 1 in one embodiment. Alternatively, in other embodiments one or more of these may be stored on a remote server/computer 130, 140 and the clinician's computer 110 may access them via LAN, WAN and/or the Internet. Such an arrangement might be preferable, for example, where the clinician uses a tablet computer 110 during consultation to maximise interaction with the patient. In this case, the clinician's computer 110 embodying one aspect of the invention may store only the user interface 280 component as an application or widget and access the other components of the system by Wi-Fi.

In another embodiment, the user interface 280 and the rules engine 270 are stored on the clinician computer 110, the patient data store 230 is stored on a server 130 for a clinic, and the knowledge base store 260 is stored on a separate server 140. Multiple clinicians' computers 110, for example within a single hospital or health provider, may access patient data held by the patient data store 230 on the server 130. In addition, clinician's computers 110 for multiple clinics or health providers may access the knowledge base store 260 held on the server 140, which is maintained and updated by a third party.

It will be apparent that various other distributions of physical and software components of the invention are possible, and each is within the scope of the present invention.

In addition, it will be apparent that the knowledge base store 260 and the rules engine 270 can be used to populate the symptoms and side effects sliders, as well as to calculate side effect risks and to map them to different drugs, to calculate a risk that different drugs will cause the patient to experience a side effect and so on.

The foregoing description has been given by way of example only and it will be appreciated by a person skilled in the art that modifications can be made without departing from the scope of the present invention.

For example, the foregoing description describes operation of the personalised medicine system 1, 100 by reference to application to the treatment of mental illness. However, the present invention is not limited to this and can be applied across the spectrum of medical complaints. Although applicable to general practice and any illness or condition, the present invention has particular benefit in the treatment of longer term illnesses and conditions, which require on-going treatment and many consultations.

Although the present invention has been described by reference to consultations and visits by the patient to the clinician, it should be understood that consultations need not be face to face (although this is preferred). Moreover, as discussed above, the personalised medicine system 1, 100 may make provision for "dummy" visits/consultations, for example involving contact of the patient with other medical professionals or for adding the results of lab tests and other investigations.

Where the words "step" and "stage" have been used in this specification, they should not be construed narrowly and, except where the context requires otherwise, they may be considered to be synonymous. Thus, the terms incorporate nodes and sub-nodes as described above. Thus, each page could be considered as being associated with a separate, individual stage or step in the workflow, with these stages or steps being grouped into nodes and higher level stages or steps.

## Claims

1. A personalised medicine system comprising:
a processor;
memory;
an input module; and
an output module,
said personalised medicine system using a knowledge base and being adapted to display pages, each page being associated with a stage in a workflow for guiding a clinical consultation, wherein:
for a plurality of said pages, advisory information based on said knowledge base is displayed depending on the stage in the workflow associated with the page.

2. A personalised medicine system according to claim 1, wherein the knowledge base comprises a plurality of sources, and a plurality of items of advisory information based on different said sources may be displayed for a said page.

3. A personalised medicine system according to claim 2, wherein a user is able to select the source on which the advisory information is based.

4. A personalised medicine system according to any one of the preceding claims, further adapted to use patient information, the displayed advisory information being further based on the patient information.

5. A personalised medicine system according to claim 4, the patient information including a time, the displayed advisory information being further based on a period elapsed since the patient information time.

6. A personalised medicine system according to claim 4 or claim 5, the patient information including patient profile data, the displayed advisory information being further based on the patient profile data.

7. A personalised medicine system according to any one of claims 4 to 6, the patient information including at least one of a rating of symptoms suffered by a patient, a rating of drug side effects suffered by the patient, a rating of the patient's adherence to a treatment regimen, a diagnosis of the patient, physical characteristics of the patient, and a treatment regimen for the patient.

8. A personalised medicine system according to any one of the preceding claims, further using a plurality of rules, said rules determining advisory information to be displayed for a said page.

9. A personalised medicine system according to claim 8, the plurality of rules including at least one scheduling rule, said scheduling rule comprising:
what advisory information is to be displayed,
a time criterion for displaying the advisory information,
a condition for resetting a clock for determining when to display the advisory information, and
a page on which to display the advisory information.

10. A personalised medicine system according to claim 9, the scheduling rule further comprising a condition for changing a timing of the advisory information based on patient profile data.

11. A personalised medicine system according to claim 9 or claim 10, further adapted to display at least one scheduling rule and to receive an input from a user for changing the time criterion.

12. A personalised medicine system according to any one of claims 8 to 11, at least one said rule for a said page comprising a hierarchy of conditions, each condition being connected with at least one other condition in the hierarchy, said patient information being used to establish whether conditions within the hierarchy are met to determine advisory information to be displayed.

13. A personalised medicine system according to any one of the preceding claims, adapted to:
determine the context in which the advisory information is to be provided;
determine evolving treatment history of the patient;
determine patient characteristics; and
depending on the determinations, provide the advisory information.

14. A method of providing advisory information in a personalised medicine system comprising:
providing access to a knowledge base;
providing access to patient information;
providing access to rules;
displaying pages, each page being associated with a stage in a workflow for guiding a clinical consultation; and
for a plurality of said pages, displaying advisory information depending on the stage in the workflow associated with the page, said advisory information being based on at least one said rule, said knowledge base and the patient information.

15. A computer program stored on a computer-readable storage medium for causing a computer to carry out a method of providing advisory information in a personalised medicine system comprising:
providing access to a knowledge base;
providing access to patient information;
providing access to scheduling rules;
displaying pages, each page being associated with a stage in a workflow for guiding a clinical consultation; and
for a plurality of said pages, displaying advisory information depending on the stage in the workflow associated with the page, said advisory information being based on at least one said rule, said knowledge base and the patient information.
